(19) Europäisches Patentamt
European Patent Office
Office européen des brevets

(11) EP 1 693 360 A1

(12) **EUROPEAN PATENT APPLICATION**
published in accordance with Art. 158(3) EPC

(43) Date of publication:
**23.08.2006 Bulletin 2006/34**

(21) Application number: **04797387.0**

(22) Date of filing: **29.11.2004**

(51) Int Cl.:
***C07C 69/013*** *(2006.01)* ***A61K 31/35*** *(2006.01)*

(86) International application number:
**PCT/CN2004/001370**

(87) International publication number:
**WO 2005/054173 (16.06.2005 Gazette 2005/24)**

(84) Designated Contracting States:
**DE FR GB**

(30) Priority: **01.12.2003 CN 200310120030**

(71) Applicant: **HUAIBEI HUIKE PHARMACEUTICAL CO., LTD.**
**235000 Anhui (CN)**

(72) Inventors:
- **YE, Hongping**
  **235000 Anhui (CN)**
- **SUN, Meng**
  **San Diego, CA 92128 (US)**

(74) Representative: **Ghioni, Carlo Raoul Maria et al**
**Bugnion S.p.A.,**
**Viale Lancetti 17**
**20158 Milano (IT)**

(54) **HUVASTATIN AND ITS PREPARATION AND FORMULATION COMPRISING THE HUVASTATIN**

(57) The invention relates to statin compounds, and it discloses novel small molecule compounds, i.e., huvastatin, which are classified into I, II, III. The invention also provides the production methods thereof and the formulations comprising the huvastatin as active components. The present compounds can be used at a lower dosage with respect to the existing statin compounds, and also can help to obtain the desired lipid levels of the patents with hyperlipidemia. Huvastatin of the invention has suitable hydrophilicity, stronger potency to reduce lipid levels, good medical effect and lower dose usage.

EP 1 693 360 A1

## Description

### FIELD OF THE INVENTION

The invention relates to statin compounds. More particularly, the invention relates to novel small molecule statin compounds, the preparation methods thereof and formulations comprising the huvastatin as active ingredients.

### BACKGROUND OF THE INVENTION

In recent years of science development, it has been well known that high blood cholesterols, lipids, and unhealthy habits such as smoking and lack of exercise, are the main contributors for causing cardiovascular diseases. Abnormal lipid level is an important risk factor of atherosclerosis and coronary heart disease. The chance of getting cardiovascular diseases and death rate caused by the above reasons has shown steady growing in recent years. According to a survey, about 10~20% of adults suffer from high total cholesterol (TC) or high triglyceride (TG), and even nearly 10% of children suffer from hyperlipemia. Therefore, it is very urgent to prevent and treat cardiovascular diseases by developing therapeutically effective drugs that can regulate blood lipid. In recent years, several new groups of cholesterol reducing drugs have been developed, of which statins have attracted much attention.

Statin drugs, which are the most effective inhibitors for the 3-hydroxyl-3-methyl glutaryl coenzyme A (HMG-CoA) reductase, are the most important discoveries of the end of the twentieth century. Since the first statin compound, lovastatin, was marketed in 1987, the 3-hydroxyl-3-methyl glutaryl coenzyme A (HMG-CoA) reductase inhibitor drugs have achieved great success clinically and profitably.

The side chain structure of statin compounds has a similar moiety as hydroxyl methyl glutaryl coenzyme A, and it can competitively inhibit the cholesterol synthesis, thus it is powerful in reducing blood lipids for patients with hyperlipemia. It is demonstrated by a series of clinical trials, especially global scale clinical trials, that using statin drugs may reduce the chance of primary and secondary coronary heart disease as well as its mortarity , which is the number one death causing disease in the world. The analysis results from a variety of research data have revealed that statins can effectively reduce the level of low-density lipoprotein cholesterol (LDL-C) and exhibit a linear positive correlation. Therefore, reducing the serum cholesterol level as greatly as possible will obtain greater clinical success. Presently, statins have entered the clinical trails to treat Alzheimer's disease and multiple sclerosis.

Statins inhibit the endogenous biosynthesis of cholesterol, and thus prevent the onset and development of atherosclerosis. They can be used to treat primary hypercholesterolemia. In addition to its cholesterol lowering aspect, statin drugs are found to be effective on treating osteoporosis, senile dementia, cardiovascular diseases, organ transplantation, stroke and diabetes. If more research and development on statins continues, statins will win the market competition.

Several statins are commercially available, such as lovastatin, simvastatin, pravastatin, fluvastatin and the like. However, pravastatin exhibits higher hydrophilicity, which indicates that the passive diffusion ability of pravastatin is lower, and as a result, its dosage is comparatively high. The hydrophilicity of simvastatin is relatively low, but low solubility also lead to relatively high dosage. Moreover, a substantial percentage of patients with hyperlipidemia cannot get their blood lipid controlled at desired level by the existing statins.

Therefore, it is urgent to develop novel and more effective compound as inhibitor for 3-hydroxyl-3-methyl glutaryl coenzyme A (HMG-CoA) reductase.

### OBJECT OF THE INVENTION

The object of the invention is to provide novel small molecule statin compounds with high potency to reduce blood lipid levels, i.e., huvastatin, which can effectively prevent and treat cardiovascular diseases

The object of the invention is also to provide the preparation methods for huvastatin. Another object of the invention is to provide the formulations comprising the huvastatin as active ingredients.

In the first aspect of the invention, there is provided a compound of structural formula (A),

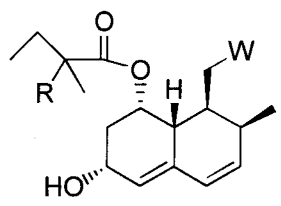

(A)

Wherein, R and W are as defined below.

In the second aspect of the invention, there is provided a pharmaceutical composition comprising an effective amount of the compound of formula (A) and a pharmaceutically acceptable carriers or excipients.

In the third aspect of the invention, there is provided the preparation methods for the compounds of formula (A). Specifically, there is provided the synthetic method for the compounds of formula (I) comprising the following steps:

Using pravastatin as starting material, the carboxylic group is protected by the formation of alkali metal salt, the 2-position of the 2-methylbutyryl group in the 8-posotion of the hydrogenated naphthalene is alkylated with alkyl halide; Or following steps are included:

Starting from pravastatin, the carboxylic group is first converted into amide, then the hydroxyl groups are protected by forming siloxanes, the 2-methylbutyryl group in the 8-posotion of the hydrogenated naphthalene is transformed into 2,2-dimethylbutyryl group with alkyl halide.

The synthetic method for the compounds of formula (II) includes the following steps:

The β-hydroxyl carboxylic acid [the product of the ring-opening reaction of the compound of formula (I)] reacts with the metal hydroxide (MOH) to afford the compound of formula (II), wherein M is lithium, sodium or potassium.

The synthetic method for the compound of formula (III) includes the following steps:

In the presence of ketone or 2,2-dialkoxylpropane, theβ ,δ -dihydroxyl carboxylic acid [the product of the ring-opening reaction of the compound of formula (I)] is converted into 6-member ring ketal catalyzed by acid, then the ketal reacts with the metal hydroxide MOH to afford the compound of formula (III), wherein M is lithium, sodium or potassium.

In the fourth aspect of the invention, there is provided the use of the compounds of formula (A) in the manufacture of a medicament for inhibiting hydroxylmethyl glutaryl coenzyme A (HMG-CoA) reductase inhibitor.

## DETAILED DESCRIPTION OF THE INVENTION

The huvastatin of the present invention, i.e., hydroxyl methyl glutaryl coenzyme A reductase inhibitor (HMG-CoA-RI) has the following general formula (A):

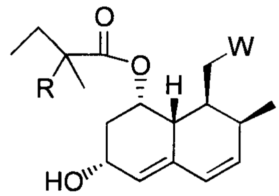

(A)

Wherein, R may be methyl, ethyl, propyl, *iso*-propyl, and butyl; W is

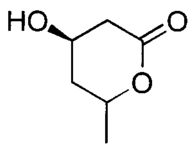

,

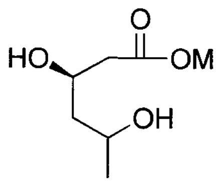

,

or

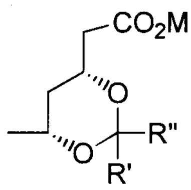
;

R' may be methyl, ethyl, propyl, *iso*-propyl, and butyl;
R" may be methyl, ethyl, propyl, *iso*-propyl, and butyl;
M is a metal ion, such as lithium, sodium, potassium and calcium.

More preferably, the huvastatin of the present invention includes the first subclass, the second subclass and the third subclass with formula (I), formula (II) and formula (III) respectively:

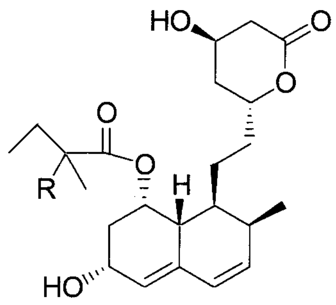

Formula (I)

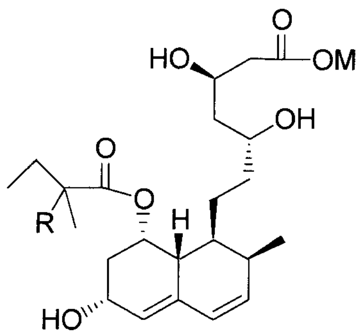

Formula (II)

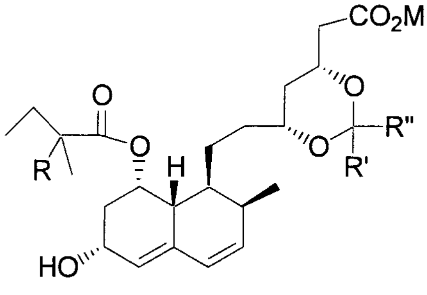

Formula (III)

Wherein, R, R', R" and M are as defined above.

Compounds 1-3 in the Example 1-3 are especially preferred, particularly the Compound 2.

Huvastatin has the same pharmacophore dihydroxyl heptoic acid moiety as the other statin compounds, and it is a semi-synthetic small molecule statin compound starting from pravastatin. Huvastatin exhibits better therapeutic effect and lower dosage due to its suitable hydrophilicity.

For all the statins, the key bioactive moiety is the chiral β-hydroxyl heptoic acid .

Huvastatin has the same pharmacophore dihydroxyl heptoic acid moiety as the other statin compounds, but the remaining structure of huvastatin is different from the other statins. It has one more hydroxyl group than simvastatin, making it more hydrophilic. The log D of Class I huvastatin determined at pH 7.4 is 0.82, lower than most other commercially available statin compounds (about 1.1~1.7). The higher hydrophilicity of huvastatin indicates the lower passive diffusion ability, and thus it is difficult to enter non-hepatic cell. However it can be absorbed by the liver via selective organic anion transportation process and can be distributed selectively and act on HMG-CoA reductase within the liver. The relative higher solubility of huvastatin in water avoids the need of higher dosage, and avoids the extensive metabolism by cytochrome P450 before being cleared; therefore the potential drug interaction of huvastatin is expected to be lowered greatly.

Class I huvastatins of the present invention and simvastatin are both inactive lactone compounds, which need to be hydrolyzed via liver to open the ring to become active inhibitor such as β-hydroxyl carboxylic acid, thus performing pharmacological action. The reaction is as follows:

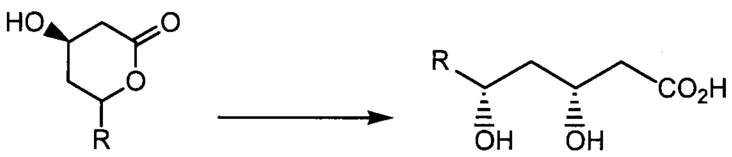

(reaction 1)

Class II huvastatins described in the present invention are active drugs. They are active (M=H) β-hydroxyl carboxylic acids, or statin derivatives that are easily to be converted to active β-hydroxyl carboxylic acid (M=Li, Na, K, or Ca). Class II huvastatin has two more hydroxyl groups and one more carboxylic group than Class I huvastatin, resulting in a higher hydrophilicity. The log D of Class II huvastatin determined at pH 7.4 is 0.42, which is comparable to that of pravastatin, but much lower than that of most other commercially available statin compounds.

In order to facilitate the preparation of formulations and increase the chemical stability of huvastatin, the present invention devises Class III huvastatins, which are relatively more stable, and much easier to be converted into active β-hydroxyl carboxylic acid compounds. They can be converted into β-hydroxyl carboxylic acid compounds catalyzed by acid inside the stomach, which is very acidic (low pH value) environment. Due to these drugs have been converted into active inhibitors before they get to the liver, the novel statins compounds not only reduce the work load of liver, but also reduce the amount of the drugs that need to be converted in the liver, thus reducing the loss resulting from metabolism. The compounds of the present invention thus have high bioavailability and lower dosage compared with known statins.

## CHEMICAL SYNTHESIS

Class I huvastatin can be prepared from pravastatin. The 2-position of the 2-methylbutyryl group in the 8-posotion of the hydrogenated naphthalene is alkylated.

One of the representative compounds is synthesized via the three reaction steps shown below.

The first step (reaction 2) is a ring-opening reaction, which transforms lactone to corresponding acyclic alkali metal salt. The reaction typically uses the solvents that can form azeotropic mixture with water easily, such as benzene, toluene and cyclohexane and the like. Equivalent amount of base is used in the reaction. Preferably potassium salt, such as potassium hydroxide, is used as a base. A small amount of C1 to C3 organic alcohol is needed in the reaction. The reaction is completed within about 1 hour. The corresponding anhydrous salt is obtained after evaporation of the solvents.

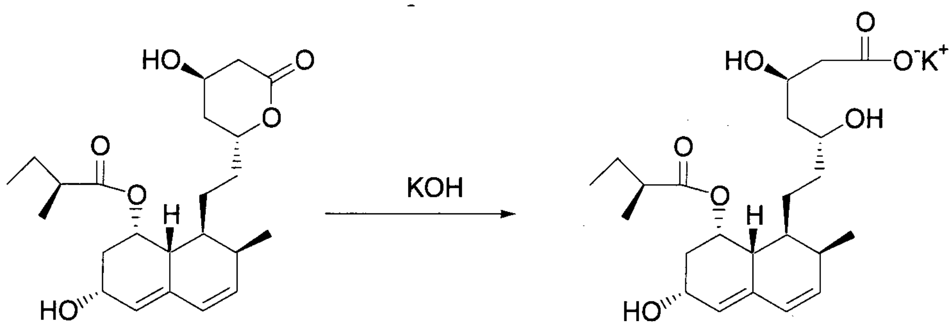


(reaction 2)

The second step (reaction3) is typically carried out in the solvents such as tetrahydrofuran, ethyl ether and butyl methyl ether, and the like. The temperature is between -50°C and -25°C. In most cases, the temperature is between -35°C and -30°C. An excess amount of base is needed in the reaction. The base used is typically metallic amine salt, such as lithium diacetamide, potassium diacetamide, sodium diacetamide, lithium diformamide, potassium diformamide, sodium diformamide, and the like. Several hours after the addition of the base, an alkyl halide such as methyl bromide or methyl iodide is added. The reaction is typically quenched with water. The reaction product is extracted at pH 3-4 by toluene, cyclohexane and the like.

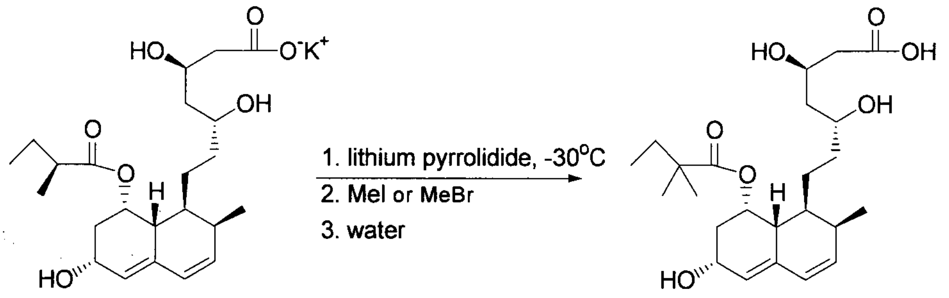


(reaction 3)

The third step of the reaction (reaction 4) yields cyclic lactone. The reaction is typically refluxed for 3-20 hours in toluene or cyclohexane. The cyclization reaction may be catalyzed by acid. The acid catalyst used in the reaction may be strong non-nucleophilic organic acids, such as toluenesulfonic acids, methanesulfonic acids, and the like; or inorganic acid, such as sulfuric acid, phosphoric acid and the like; or acidic ion-exchange resin and the like.

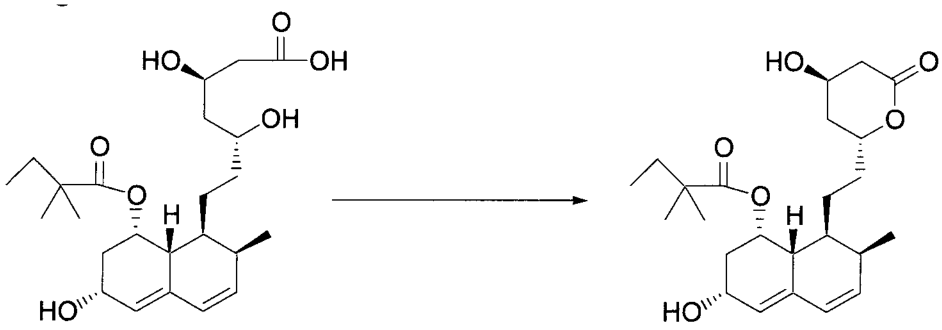

(reaction 4)

The other synthetic methods for huvastatin are shown in the following reactions 5-11:

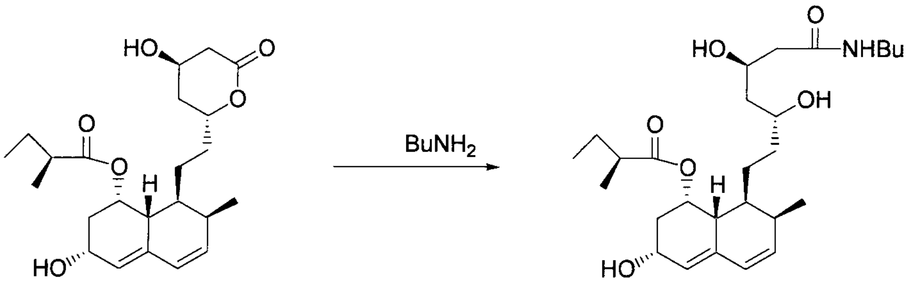


(reaction 5)

The protection of the carboxylic group: by reaction with organic the lactone ring is opened to form the corresponding amide.

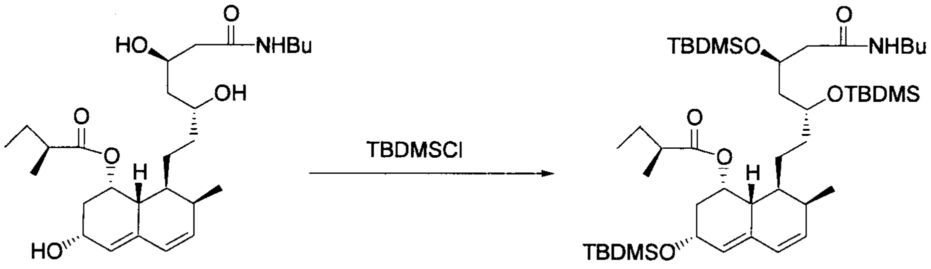


(reaction 6)

The protection of the hydroxyl group: all of the hydroxyl groups are transformed into the corresponding siloxane, such as tert-butyl dimethyl siloxane.

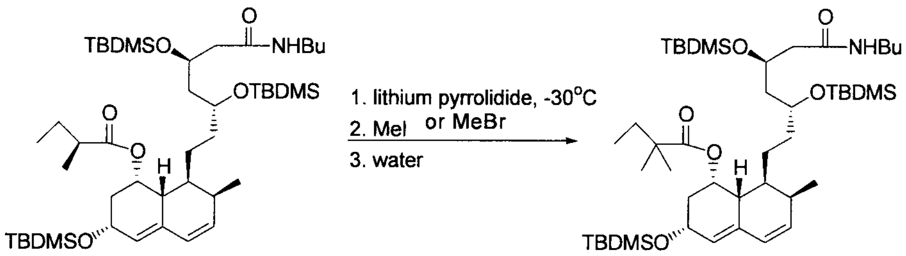


(reaction 7)

The 2-position of the 2-methylbutyryl group in the 8-posotion of the hydrogenated naphthalene is methylated.

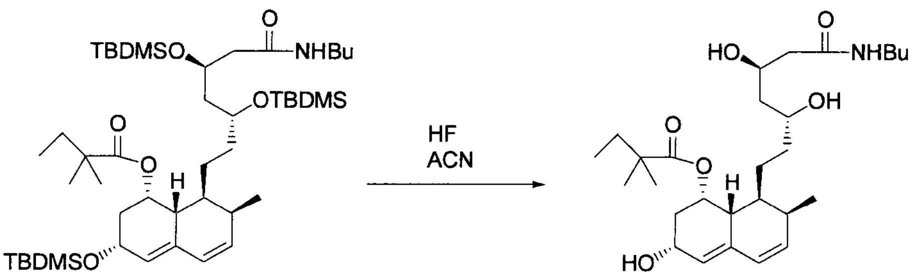


(reaction 8)

The deprotection of the hydroxy group: all the protected hydroxyl groups by the tert-butyl dimethylsiloxane are transformed into the corresponding hydroxyl groups.

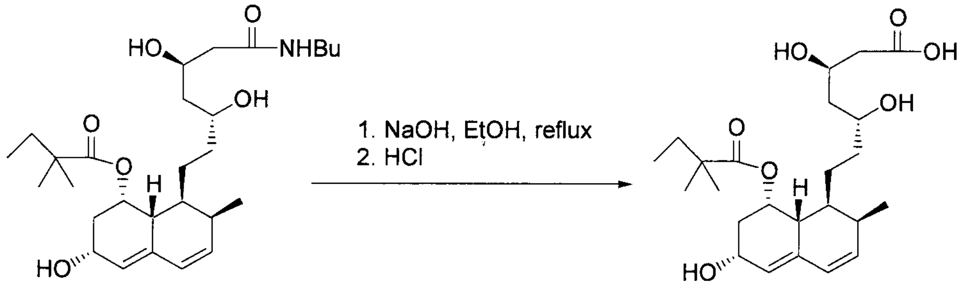


(reaction 9)

The deprotection of the carboxylic group: the amide is transformed into corresponding carboxylic group.

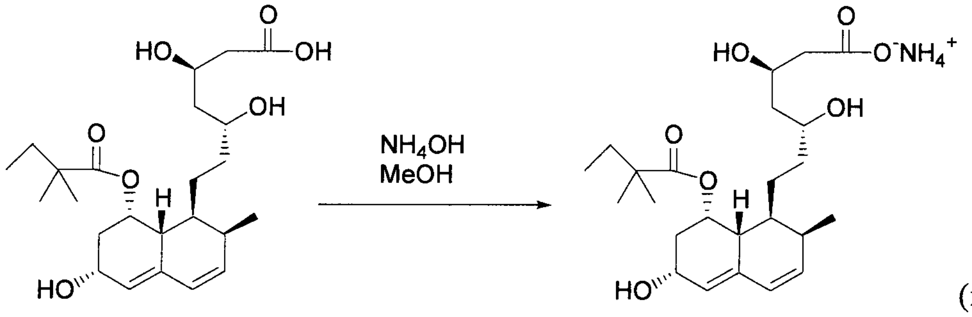


(reaction 10)

The carboxylic group is transformed into the corresponding ammonium salt in order to facilitate the recrystallization and purification.

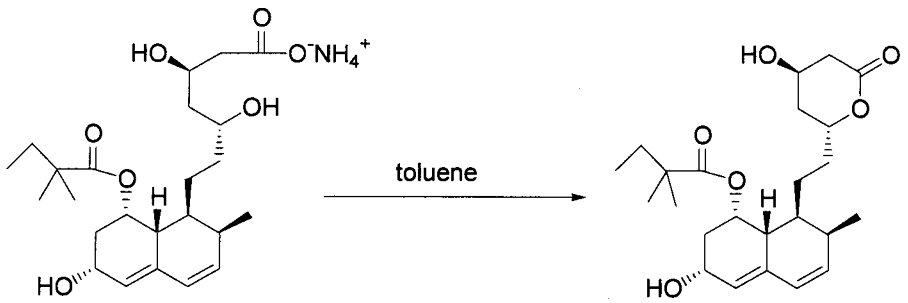


(reaction 11)

Intra-annular esterification reaction affords the corresponding Class I huvastatin, i.e., 2,2-dimethylbutyricacid-3-hydroxy-8-[2-(4-hydroxy-6-oxo-2-tetrahydro pyranyl)-ethyl]-7-methyl-1,2,3,7,8,8a-hexahydronaphthalen-1-yl ester.

Class II huvastatin may be synthesized via one step of reaction shown below.

The salification reaction is achieved by the reaction of β-hydroxy carboxylic acid (the product of ring-opening reaction of the Class I huvastatin) with a corresponding base.

The corresponding base may be lithium hydroxide, sodium hydroxide and potassium hydroxide. The calcium salt can be obtained through cation exchange, e.g.,via precipitation with calcium chloride

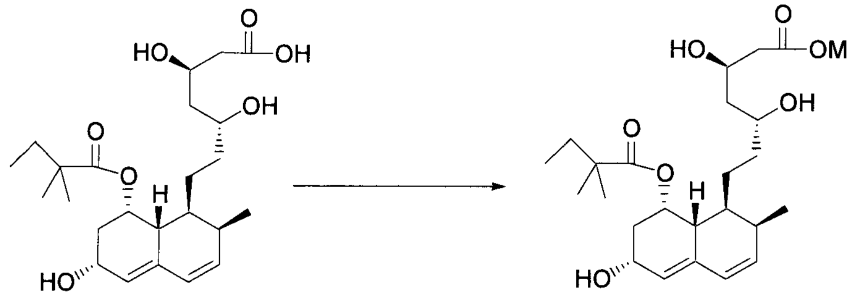

(reaction 12, R=$CH_3$ as an example)

Class III huvastatin may be synthesized via two-step reactions shown below.

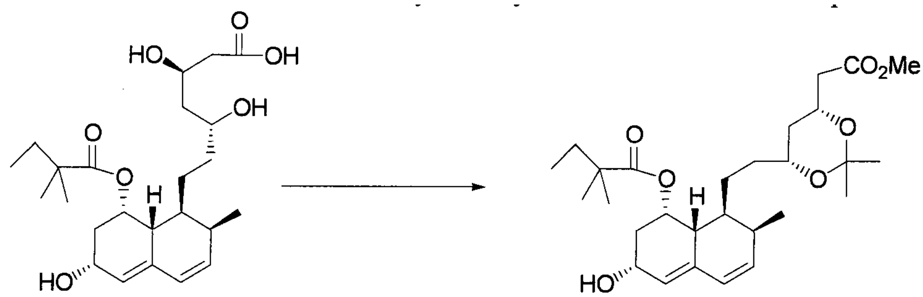

(reaction 13, R=$CH_3$ as an example)

2,2-dimethylbutyricacid-3-hydroxy-8-[2-(6-methoxycarbonyl-2,2-dimethyl[1, 3]-dioxanyl)-ethyl]-7-methyl-1,2,3,7,8,8a- hexahydronaphthalen-1-yl ester.

The first step is to convert theβ ,δ -dihydroxyl group of the starting material to 6-member ring ketal, meanwhile the carboxylic group of the molecule is converted to the corresponding ester. The reagent suitable for this step may be the corresponding ketone, or the corresponding 2, 2-dialkoxypropane. The alkoxy of the 2,2-dialkoxypropane is $C_1$ to $C_3$ alkoxy group, such as 2,2-dimethoxypropane, 2,2-diethoxypropane, 2-methoxy-2-ethoxypropane, 2,2-dipropoxypropane, and the like. The corresponding ketone may be used as the staring material, such as acetone, methyl ethyl ketone and pentanone, and the like.

The catalyst used in the reaction may be strong non-nucleophilic organic acids, such as *p*-toluenesulfonic acids, methanesulfonic acids, and the like; or inorganic acid, such as sulfuric acid, phosphoric acid and the like; or acidic ion-exchange resin and the like.

This step of the reaction may use the corresponding reagent directly as solvent, such as 2,2-dimethoxypropane, and the like. Or the organic compounds such as toluene, dichloromethane, and dichloroethane may be used as solvent. Under the conditions, the ratio of the reagent to the starting lactone is typically between 1:1 and 2:1.

The temperature in this step is typically between -20°C to 60°C, in most cases between 0°C to 30°C. The reaction is performed under inert gas such as nitrogen. The amount of the catalyst used in the reaction is between 0.1mol% and 100mol%, typically between 1mol% and 5mol%.

The second step is the hydrolysis catalyzed by a base. The hydrolysis reaction needs at least one equivalent of base, typically between 1 and 1.5 equivalents of base. The temperature is between -20°C to 60°C, typically between 0°C to 30°C. The solvent may be water, alcohol such as methanol and ethanol, toluene, or acetone and the like, or the combination of water and other organic solvents. When mixed solvents are used, phase transfer catalyst is needed in the reaction.

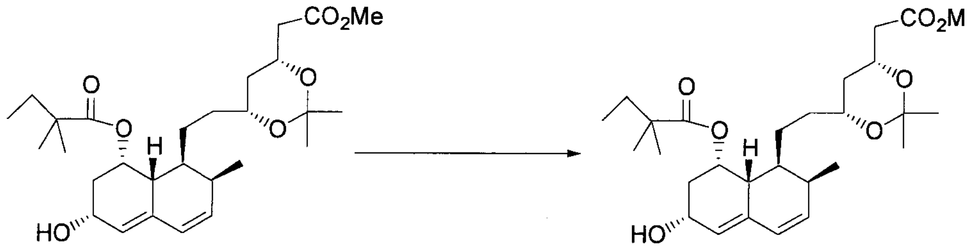

(reaction 14, R=$CH_3$ as an example)

Class III huvastatin compounds are acid-labile, thus the formulation of these statin compounds need to be stabilized with necessary buffering agents. The compounds that are suitable to be used as buffering agents may be organic base, such as amino sugar and the like; inorganic base, such as sodium carbonate, sodium bicarbonate, disodium hydrogen phosphate and the like; organic salts, such as sodium acetate, and the like; metal oxide, such as magnesium oxide; amino acid, such as arginine, and the like.

The inhibiting constant $K_i$ of statin with HMG-CoA is determined using conventional methods. $K_i$ is the binding (inhibiting) constant of statin compounds with 3-hydroxyl-3-methyl glutaryl coenzyme A (HMG-CoA). As shown in the table below, huvastatin has the smallest $K_i$ compared with the other statins. For statin compounds, smaller Ki means stronger inhibiting effect for HMG-CoA. Thus huvastatin is a suitable drug compound for reducing lipid levels and lowering cholesterol.

$$E+I \quad = \quad (E \cdot I) \quad = \quad (EI)$$

metastable state      stable state

The results are tabulated as below:

| Name | Lovastatin | Simvastatin | Pravastatin | Atorvastatin | Huvastatin (formula II) |
|---|---|---|---|---|---|
| $K_i$ | 51 | 12 | 43 | 8.2 | 7.3 |

As shown in the above table, huvastatin (formula II) has smaller inhibiting constant, and its inhibiting effect is stronger than that of atorvastatin.

**FORMULATIONS OF THE PREPARATION**

The invention provides a pharmaceutical composition comprising 0.01-99.9 wt% of compounds of formula (A) or the pharmaceutically acceptable salts thereof together with a pharmaceutically acceptable carriers or excipients.

The dosage form of the present invention is not particularly limited. Formulation for other statin compounds, such as capsule formulation and tablet formulation are suitable for huvastatin of the present invention. Typically, the formulation includes 1.0 to 10.0 mg active pharmaceutical ingredient and a pharmaceutically acceptable carrier or excipients. The total amount of the active pharmaceutical ingredient and lactose may be varied 0.1-100 mg.

Take tablet formulation as an example. Generally speaking, it is prepared by blending fixed ratio of the active pharmaceutical ingredient, fillers, lubricants, binding agents, disintergrants, and buffering agents, scrunching evenly (the temperature is typically controlled below 50 °C), and then pressed into tablets.

The pharmaceutically acceptable carriers or excipients described above refers to the conventional pharmaceutical carriers or excipients, for example:

The fillers may be lactose, spray dried anhydrous lactose, microcrystalline cellulose, powdered cellulose, calcium sulphate, and the like.

The disintergrants may be low substituted hydroxpropyl cellulose, crosslinked croscarmellose sodium, gelated silica gel, sodium hydroxymethyl starch, microcrystalline cellulose, crosslinked polyvinyl-pyrrolidone, crosslinked sodium

carboxymethyl cellulose, sodium carboxymethyl starch, and the like.
The binding agents may be maltodextrin, pre-gelated starch, polyvinyl-pyrrolidone (PVP), vinyl-pyrrolidone and the copolymer of vinylacetate (KOLLIDON VA64), and the like.
The lubricants may be micro powder silica gel, magnesium stearate, or calcium stearate.

## DRUG ADMINISTRATION

Unless otherwise indicated, the compounds and pharmaceutical composition of the present invention may be administrated orally; topically; parenterally such as intramuscular injection, intravenous injection or subcutaneous injection; or by spray inhalation. Oral administration is preferred.
When the compound of the invention is administrated orally in the form of tablets or capsules, the dosage ranges from about 1mg to 1000mg for an adult with the average body weight of 60-70 kilogram. When the compound of the invention is administrated parenterally in the form of injection, the dosage may range from about 0.1mg to 500mg, and may be administrated once or several times a day. The unit dosage of the pharmaceutical composition generally includes 1mg-500mg of active pharmaceutical ingredient, typically 1mg, 5mg, 10mg, 25mg, 50mg, 100mg, 200mg, 300mg, 400mg, 500mg of active pharmaceutical ingredient.
When the composition of the invention is used for the treatment of a specific disease, the specific dose level and administration scheme of the active pharmaceutical ingredient will depend upon a variety of factors including body weight, age, sex, the inevitable medical symptom, the severity of the particular disease, route of drug administration, frequency of administration, ...etc.
The following examples illustrate particular methods for preparing compounds in accordance with this invention. These examples are illustrative and are not to be read as limiting the scope of the invention as it is defined by the claims.

### Example 1: Preparation of Class-I Huvastatin (Compound 1)

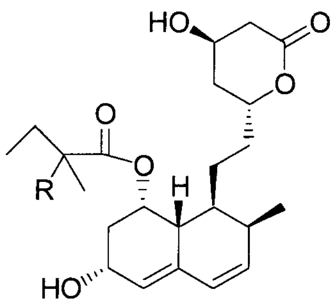

R=methyl

2,2-Dimethylbutyricacid-3-hydroxy-8-[2-(4-hydroxy-6-oxo-tetrahydropyran-2-yl)-et hyl]-7-methyl-1,2,3,7,8,8a-hexahydronaphthalen-1-yl ester
Pravastatin (12.25 mmol) was dissolved in cyclohexane (100 ml) and 2-propanol (12 ml) at room temperature under dry nitrogen, then added pre-prepared 4.91M potassium hydroxide aqueous solution (2.5 ml) into the above pravastatin solution in one portion. The reaction solution was stirred for about 0.5-1hour at room temperature under dry nitrogen. The solid product obtained by evaporation of solvents was re-dissolved in anhydrous cyclohexane (150 ml). The solution was concentrated to a volume of 15ml, then diluted with 35 ml anhydrous THF. The prepared potassium carboxylate solution was cooled down to -35°C.
A solution of lithium pyrrolidine (3.6 ml) in anhydrous THF (30ml) was cooled to -5°C and a solution of 1.55 M n-butyllithium was gradually added in with the internal temperature maintained below 0°C during the addition. The lithium pyrrolidide was slowly added to the cooled solution of pravastatin potassium salt, maintaining the internal temperature below -30°C throughout the addition. Two hours later, a solution of methyl bromide (2.36ml) was added slowly with an internal temperature maintained below -20°C. This reaction mixture was maintained at this temperature for 1 hour.
1.55 M n-butyl lithium solution (12 ml) was added into a solution of pyrrolidine (1.6 ml) in THF (15 ml), the internal temperature maintained below 0°C. Lithium pyrrolidide solution produced in this way was gradually added to the reaction mixture maintaining an internal temperature below -30°C.
The reaction mixture was aged between -35°C and -30°C for one hour, then another portion of the methyl

bromide solution (3.01g) was added slowly maintaining an internal temperature below -20°C. The reaction mixture was aged at this temperature for one hour.

The mixture was transferred into 100 ml of water. The layers were separated and the aqueous layer was adjusted to pH 4.5 with 20% phosphoric acid. The acidified aqueous phase was extracted with cyclohexane (3 X 300 ml), and combined cyclohexane extracts were washed twice with water, and then dried with anhydrous sodium sulfate.

The solution was filtered and slowly concentrated to a volume of 40 ml, then cooled down to room temperature. The crude solid product was obtained after filtration, and the pure product of Class I Huvastatin was re-crystallized from methanol and water.

m.p=162-165°C (decomp),

HNMR ($CDCl_3$, 400MHZ, $\delta_{ppm}$): 0.86(d, 3H), 0.88(t, 3H), 1.11(s, 3H), 1.12(s, 3H), 1.2-2.5(m, 13H), 3.37(br, 1H), 3.51 (br, 1H), 3,64(m, 1H), 4.08(m, 1H), 4.26(m, 1H), 5.31(br, 1H), 5.52(br, 1H), 5.92(dd, 1H), 5.99(d, 1H).

The inhibiting constant ($K_i$) of Compound 1 with HMG-CoA is 14.6 (Holdgate, G.GA; Ward, W.H.J.; McTaggart, F., *Biochem. Soc. Trans.* **2003**, *vol. 32*, 528-531).

**Example 2: Preparation of Class-II Huvastatin potassium salt (Compound 2)**

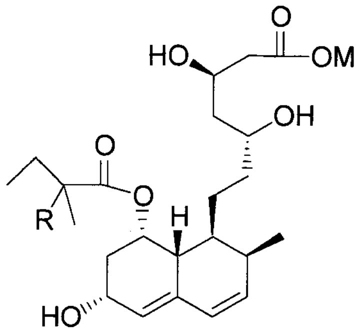

R=methyl, M=potassium

100 mmol of the ring-opening product of Class I Huvastatin (Compound 1) was suspended in cyclohexane (100 ml) and 2-propanol (12 ml) at room temperature under dry nitrogen, then 101 ml of 4.91 M potassium hydroxide aqueous solution was gradually added into above solution. The reaction mixture was stirred for a period of 30 to 60 minutes at room temperature under dry nitrogen. Crude solid product was obtained after distilling off solvents. The crude product was suspended in toluene and was dried through azeotropic distillation. The solid product obtained was washed with cold acetone, and then dried under vacuum to give Compound 2.

m.p=168-171°C (decomp),

HNMR ($CDCl_3$, 400MHz, $\delta_{ppm}$): 0.86(d, 3H, 2CH3), 0.89(t, 3H, 3'-$CH_3$), 1.11(s, 3H, 2'-$CH_3$), 1.12 (s, 3H, 2'- $CH_3$), 1.2-2.5 (m, 14H), 3.38(br, 1H,OH), 3.53(br, 1H,OH), 3,65(m, 1H,δH), 4.09(m, 1H,βH), 4.26(m, 1H, 6-H), 5.33(br, 1H, 8-H), 5.52 (br, 1H, 5-H), 5.91(dd, 1H, 3-H), 6.01(d, 1H, 4-H).

The inhibiting constant ($K_i$) of Compound 2 with HMG-CoA is 7.3 (Holdgate, G.GA; Ward, W.H.J.; McTaggart, F., *Biochem. Soc. Trans.* **2003,** *vol. 32,* 528-531).

**Example 3: Preparation of Class III Huvastatin (Compound 3)**

Step 1: The reaction is shown below:

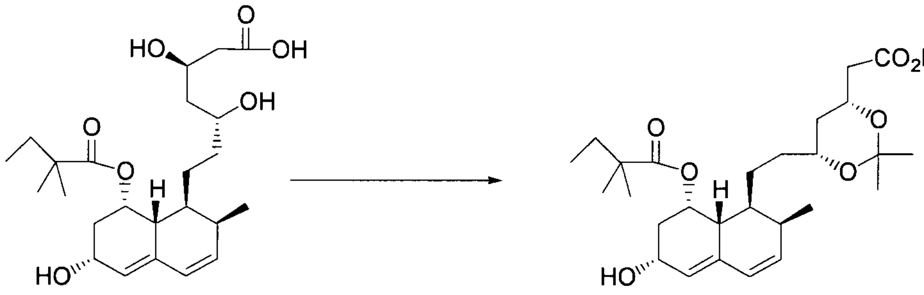

(reaction 15)

100 mmol of Class-I Huvastatin carboxylic acid compound was suspended in 50 ml 2,2-dimethoxypropane at room temperature under dry nitrogen, and the mixture was stirred for more than ten minutes till all the starting materials distributed uniformly in the solution. 5 mmol of catalyst p-toluene sulphonic acid was added to this reaction mixture, and the reaction mixture was stirred at room temperature under dry nitrogen until all solids were dissolved and clear solution was obtained. After stirring at room temperature for another 30 minutes, 50 mmol of sodium carbonate was added, and kept stirring for another one hour. After filtration and rotary evaporation most of the solvents was removed under reduced pressure, the residue was separated with 200 ml ethyl acetate and 100 ml saturated sodium chloride aqueous solution. The isolated ethyl acetate phase was dried with anhydrous sodium sulfate. The 1,3-dioxane ester compound was obtained after ethyl acetate was distilled off.

Step 2: Formation of 1,3-dioxane sodium carboxylate

The reaction is shown by the equation below (M is sodium ion)

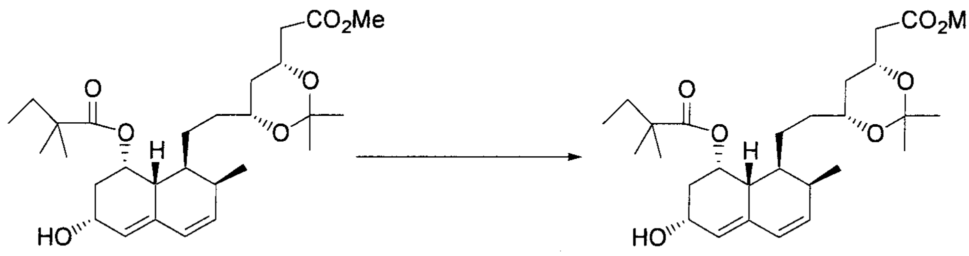

100 mmol of 1,3-dioxane ester compound was suspended in 200 ml distilled water. To this suspension, 50% sodium hydroxide aqueous solution was gradually added until homogeneous solution was obtained. The solution pH was adjusted and then maintained at 12 for one hour, after that the pH was adjusted to pH 10 with hydrochloric acid. The reaction mixture was extracted with ethyl acetate, and the ethyl acetate extract was concentrated to obtain solid crude product. The crude product was suspended in toluene and dried through azeotropic distillation. The solid product obtained was washed with cold acetone, then dried under vacuum to obtain compound 3.
m.p. = 46-49°C (decomp),
HNMR ($CDCl_3$, 400MHZ, $\delta_{ppm}$): 0.86(d, 3H), 0.88(t, 3H), 1.11(s, 3H), 1.13(s, 3H), 1.2-2.6(m, 13H), 1.37(s, 3H), 1.45(s, 3H), 3.47(br, 1H), 3,66(m, 1H), 4.09(m, 1H), 4.27(m, 1H), 5.32(br, 1H), 5.52(br, 1H), 5.93(dd, 1H), 5.97(d, 1H).

The inhibiting constant ($K_i$) of Compound 3 with HMG-CoA is 17.7 (Holdgate, G.GA; Ward, W.H.J.; McTaggart, F., *Biochem. Soc. Trans.* **2003,** *vol. 32*, 528-531).

**Example 4:**

Tablets of 100 mg/tablet comprising 0.5 mg active ingredients were prepared by mixing the following components using conventional methods.

| Ingredients | Required amount (mg) |
|---|---|
| Active component (Compound 1, 2 or 3) | 5.0 |

(continued)

| Ingredients | Required amount (mg) |
|---|---|
| Lactose | 42.0 |
| Microcrystalline cellulose | 30.0 |
| Cellulose powder | 16.0 |
| Polyvinyl-pyrrolidone | 0.5 |
| Crosslinked Croscarmellose Sodium | 5.0 |
| Glucosamine | 0.5 |
| Magnesium stearate | 1.0 |
| Total | 100 |

### Example 5: Animal Test

Compound 1, 2 or 3 prepared in examples 1-3 were administered to rats (10 rats in each group), respectively, twice per day, 0.05 mg/dose/rat or 0.1 mg/dose/rat. Blood samples were taken and analyzed after one week for blood lipid level and cholesterol.

The results has revealed that blood lipid of groups receiving Compound 1, 2 or 3 is lower than that of the control group. Compound 2 has the best lipid-lowering effect.

While the invention has been described by references to specific embodiments, this was for the purpose of illustration only. Numerous alternative embodiments will be apparent to those skilled in the art and are considered within the scope of these claims.

## Claims

1. A compound as shown in formula (A):

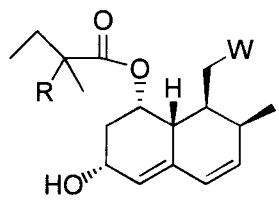

(A)

wherein,
R is methyl, ethyl, propyl, *iso*-propyl, or butyl;
W is

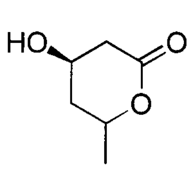 , 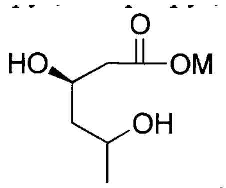 ,

or

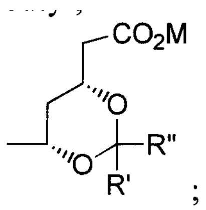

R' is methyl, ethyl, propyl, *iso*-propyl, or butyl;
R" is methyl, ethyl, propyl, iso-propyl, or butyl; and
M is a metal ion.

**2.** The compound of Claim 1 having the following formula:

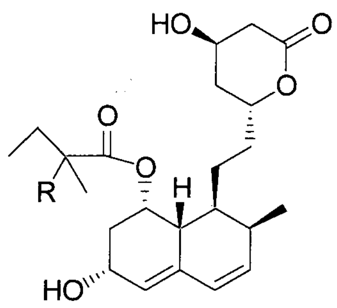

Formula (I)

wherein R is as defined above.

**3.** The compound of Claim 1 having the following formula:

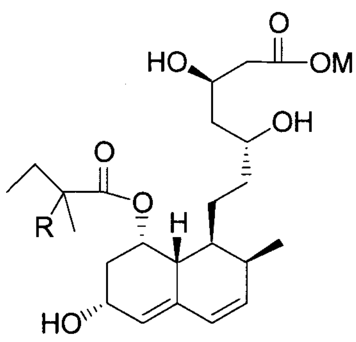

Formula (II)

wherein R is as defined above; and
M is lithium, sodium, potassium or calcium.

**4.** The compound of Claim 1 having the following formula:

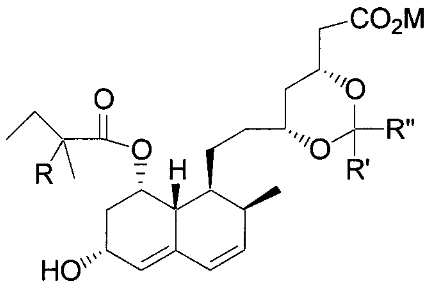

Formula (III)

wherein, R, R', and R" are as defined above; and
M is lithium, sodium, potassium or calcium.

**5.** The compound of Claim 1, wherein the compound is selected from the group consisting of:

Compound 1: 2,2-dimethylbutyricacid-3-hydroxy-8-[2-(4-hydroxy-6-oxo-tetrahydro pyran-2-yl)-ethyl]-7-methyl-1,2,3,7,8,8a-hexahydronaphthalen-1-yl ester;
Compound 2: the compound of formula (II), wherein R=methyl, M=K;
Compound 3: the compound of formula (III), wherein R=R'=R"=methyl, M=K.

**6.** A pharmaceutical composition comprising an effective amount of the compound of formula (A) and a pharmaceutically acceptable carrier.

**7.** The synthetic method of the compound of formula (I), wherein the method comprises the steps of:

starting from pravastatin, after the protection of the carboxylic group with formation of alkali metal salt, the 2-position of the 2-methylbutyryl group in the 8-posotion of the hydrogenated naphthalene is alkylated with alkyl halide;
or the method comprises the following steps:

starting from pravastatin, after the carboxylic group is converted into amide and the hydroxyl group is protected by siloxane, the 2-methylbutyryl group in the 8-posotion of the hydrogenated naphthalene is transformed into 2,2-dimethylbutyryl group with alkyl halide.

**8.** The synthetic method of the compound of formula (II), comprising the steps of:

reacting β-hydroxyl carboxylic acid, i.e., the product of the ring-opening reaction of the compound of formula (I), with a base of formula MOH, thereby forming the compound of formula (II), wherein M is lithium, sodium or potassium.

**9.** The synthetic method of the compound of formula (III), comprising the steps of:

in the presence of ketone or 2,2-dialkoxylpropane, converting the β , δ -dihydroxyl carboxylic acid, i.e., the product of the ring-opening reaction of the compound of formula (I), into 6-member ring ketal by acid catalysis, and reacting the ketal with the base of formula MOH, thereby forming the compound of formula (III),

wherein M is lithium, sodium or potassium.

**10.** A use of the compound of formula (A) in the manufacture of drugs for inhibiting hydroxylmethyl glutaryl coenzyme A reductase.

# INTERNATIONAL SEARCH REPORT

International application No.
PCT/CN2004/001370

A. CLASSIFICATION OF SUBJECT MATTER

C07C69/013, A61K31/35

According to International Patent Classification (IPC) or to both national classification and IPC

B. FIELDS SEARCHED

Minimum documentation searched (classification system followed by classification symbols)

C07C,A61K

Documentation searched other than minimum documentation to the extent that such documents are included in the fields searched

Electronic data base consulted during the international search (name of data base and, where practicable, search terms used)

WPI,EPODOC,PAJ,CNKI,CA,CPRS

C. DOCUMENTS CONSIDERED TO BE RELEVANT

| Category* | Citation of document, with indication, where appropriate, of the relevant passages | Relevant to claim No. |
|---|---|---|
| A | US4346227A, 24.August.1982, see the whole document | 1-10 |
| A | US444784A, 24.April.1984, see the whole document | 1-10 |
| A | CN1232030A, 20.October.1999, see the whole document | 1-10 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

Date of the actual completion of the international search
29.January.2005

Date of mailing of the international search report
24 · MAR 2005 (2 4 · 0 3 · 2 0 0 5)

Name and mailing address of the ISA/
6 Xitucheng Rd., Jimen Bridge, Haidian District,
100088 Beijing, China
Facsimile No. (86-10)62019451

Authorized officer
WU HONGQUAN 吴汉 印红
Telephone No. 86-10-62085611

Form PCT/ISA /210 (second sheet) (January 2004)

**INTERNATIONAL SEARCH REPORT**
Information on patent family members

International application No.
PCT/CN2004/001370

| | | | |
|---|---|---|---|
| US4346227A | 19820824 | GB2077264A | 19811216 |
| | | BE889150A | 19811209 |
| | | DE3122499A | 19811224 |
| | | FR2483912A | 19811211 |
| | | SE8103560A | 19820104 |
| | | NL8102737A | 19820104 |
| | | DK8102470A | 19820111 |
| | | JP57002240A | 19820107 |
| | | FI8101762A | 19820129 |
| | | JP57050894A | 19820325 |
| | | JP57108039A | 19820705 |
| | | JP57067575A | 19820424 |
| | | US4346227A | 19820824 |
| | | CA1150170A | 19830719 |
| | | AT8102567A | 19830915 |
| | | US4410629A | 19831018 |
| | | GB2077264B | 19840426 |
| | | US4448979A | 19840515 |
| | | CH655090A | 19860327 |
| | | JP61013699B | 19860415 |
| | | DE3122499C | 19871126 |
| | | JP62054476B | 19871116 |
| | | SE453389B | 19880201 |
| | | JP63021672B | 19880509 |
| | | IT1144598 | 19861029 |
| | | JP63048858B | 19880930 |
| | | NL191738C | 19960102 |
| US4444784A | 19840424 | None | |
| CN1232030A | 19991020 | IL138119 A | 20040512 |
| | | EP0940395 A1 | 19990908 |
| | | WO9945003 A1 | 19990910 |
| | | NO9901045 A | 19990906 |
| | | AU2861299 A | 19990920 |
| | | US6100407 A | 20000808 |
| | | NO200004357 A | 20001106 |
| | | EP1064275 A1 | 20010103 |
| | | CZ200003194 A3 | 20010411 |

Form PCT/ISA /210 (patent family annex) (January 2004)